# EUROPEAN PATENT SPECIFICATION

(11) **EP 2 509 586 B1**
(45) Date of publication and mention of the grant of the patent: **02.05.2018**
(21) Application number: 10792884.8
(22) Date of filing: 09.12.2010
(51) Int. Cl.: A61K 9/20, A61K 31/00, A61K 47/36, A61K 31/44

(54) **MUCOADHESIVE BUCCAL TABLETS FOR THE TREATMENT OF OROFACIAL HERPES**
MUKOADHÄSIVE BUKKALE TABLETTEN ZUR BEHANDLUNG VON OROFAZIALEM HERPES
COMPRIMÉS BUCCAUX MUCOADHÉSIFS POUR LE TRAITEMENT DE L'HERPÈS OROFACIAL

(30) Priority: 09.12.2009 EP 09290925; 09.12.2009 US 634225
(43) Date of publication of application: 17.10.2012
(73) Proprietor: VECTANS PHARMA, 75009 Paris (FR)
(72) Inventor: ATTALI, Pierre, F-94300 Vincennes (FR); COSTANTINI, Dominique, F-75014 Paris (FR); LEMARCHAND, Caroline, F-75014 Paris (FR)
(74) Representative: Ipside
(86) International application number: PCT/EP2010/069313
(87) International publication number: WO 2011/070125

(56) References cited:
- WO-A1-01/82905
- WO-A1-95/15137
- WO-A2-2009/049648
- US-A1- 2003 108 603
- US-A1- 2009 169 511
- DE VRIES M E ET AL: "DEVELOPMENTS IN BUCCAL DRUG DELIVERY", CRITICAL REVIEWS IN THERAPEUTIC DRUG CARRIER SYSTEMS, BEGELL HOUSE PUBLISHING INC, vol. 8, no. 3, 1 January 1991 (1991-01-01) , pages 271-303, XP002044702, ISSN: 0743-4863

## Description

### TECHNICAL FIELD

The present invention relates to the treatment and/or prevention of muco-cutaneous herpes simplex virus diseases using prolonged release mucoadhesive buccal tablets comprising an acyclic guanosine antiviral agent. These tablets are particularly suitable for the treatment and/or prevention of orofacial herpes disease.

### BACKGROUND OF THE INVENTION

Herpes Simplex Virus (HSV) is the most widely spread virus in the herpes family. It has been identified as the pathogenic agent in a number of infectious processes involving the muco-cutaneous tissues, the nervous central system, and in severe cases, the viscera. About one billion people worldwide have been infected with herpes viruses which belong to two types according to the antigenic differences in the envelope proteins: HSV type 1 (HSV-1) and HSV type 2 (HSV-2). The largest reservoir of HSV is associated with herpes labialis infection, most commonly resulting from primary infection with HSV-1 during childhood. The prevalence of HSV-1 and HSV-2 is estimated to be between 50-95% and 6-50% respectively, depending on several factors such as age, race, sex, marital and social status. HSV-1 primarily causes oral infections, whereas HSV-2 is the most common cause for genital ulcers.

Herpes labialis (also known as "orofacial herpes", "orolabial herpes", "cold sores" or "fever blisters") is the most common recurrent disease caused by HSV-1 infection with considerable incidence (85% of the world's population is seropositive for HSV-1). In many cases, it causes blisters or sores on or around the mouth that are commonly known as cold sores or fever blisters. Hence, it is aesthetically unpleasant and induces considerable discomfort to the patient. Sores associated with herpes labialis typically heal within 2-3 weeks, but the virus that causes them is not removed from the body. Recurrent episodes of herpes labialis are frequent. That means that the infected herpes virus becomes dormant in the facial nerves, following orofacial infection, and periodically reactivates to create sores in the same area of the mouth or face that the original infection occurred.

It is estimated that about 20-40% of people have experienced orofacial herpes at some time. In Europe, the overall incidence is approximately 3 per 1000 people per year and more than 10 per 1000 people per year in those aged over 80 years. In the United States, approximately 130 million individuals over 12 years are infected with HSV-1. Approximately one third of the patients with HSV-1 infection will experience recurrent episodes of herpes labialis.

Most people acquire HSV-1 asymptomatically. Primary infection usually occurs in childhood. It is usually asymptomatic, but may present in different forms: transient gingivostomatitis, pharyngitis or other lesions in and the oral cavity, sometimes with fever.

HSV infects mucous membranes, replicates at the cutaneous entry site (cells of the epidermis and dermis) and may infect the sensory nerve ending that innervate cells at the initial infection site provided that the number of virions is sufficient. The virus is then transported to the sensory nerve nuclei, the trigeminal ganglion for herpes labialis, and remains in a latent state in sensory neurons for the life of the host cell. HSV reactivation could occur in a fraction of neurons population harbouring the latent virus. Upon reactivation, HSV could disseminate throughout the axon from infected cells to the mucosa. Hence, as HSV remains in the mammalian body, orofacial herpes is a recurrent disease. That means that, following reactivation of latent HSV, patients undergo many occurrences of herpes labialis throughout their life. A main challenge for current herpes labialis treatment is to lower the recurrence, that means to lower the frequency, duration, and severity of these occurrences. The reservoir site of persistent HSV, from the primary infection until reactivation and in between recurrences was found to be in the dorsal roots of the trigeminal ganglion or in the sensory root ganglion. However, the triggering of the viral genome in the ganglion does not exclusively explain the recurrences of herpes labialis. Other sites can be infected with HSV and constitute a reservoir site of persistent HSV. It was suggested in the state of the art that HSV may well be occult in epithelial cells. Hence, mucous and skin cells might also act as a preferential site for latent HSV (Zakay-Rones Z. and al., Microbiologica, 1986 ; N. Hochman and al., Israel Journal of Dental Sciences, 1989). Moreover, the viral load in saliva during herpes labialis occurrences increases the persistence of HSV. Indeed, high concentrations of virions in saliva lead to reinfestations through the mucous membranes of the mouth. Such infections further supply the HSV reservoir and thus the latency of HSV.

Up to now no herpes labialis treatment acts on the reservoir of latent HSV.

Reactivation may be triggered by several factors that are specific to the patient, i.e., emotional stress, fever, exposure to ultraviolet light, menses, premenstrual tension, surgical (such as dental or neural) procedures, lip tattooing, dermabrasion or oral traumas. Immune suppression also favours reactivation.

The clinical manifestations of herpes labialis infection depend largely on the anatomic site of infection, immune status of the host and the antigenic type of virus. Most lesions occur on the lips. However, lesions can also occur on the nose, cheeks, or chin. Lesions occurring in the oral cavity or face are less common. Intra-oral lesions are hard to locate and are difficult to distinguish from apthous ulcers, oropharyngeal candidiasis or cancer sores.

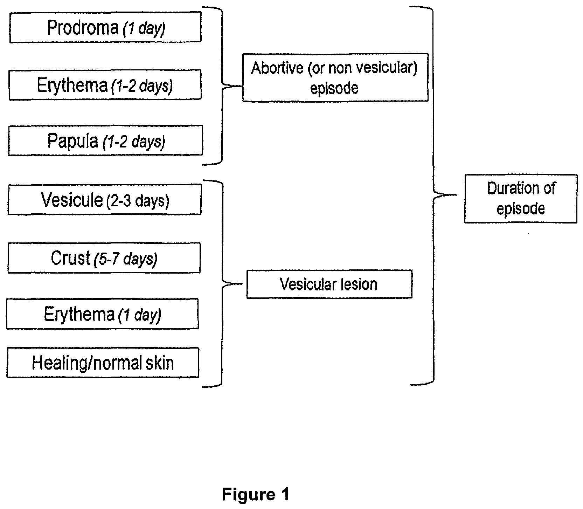

In the immunocompetent population, occurrences of herpes labialis follows a predictable course (schematized in Figure 1), called episodes. An episode of herpes labialis comprises:
- Prodrome: episodes of herpes labialis begin with a prodromal stage, during which patients experience pain, burning, itching, tingling, or discomfort in the area of the lesion. It is thus the patient's first indication that herpes labialis is developing. The prodromal stage reliably predicts the onset of lesion outbreak.
- Erythema: erythema, a redness of the skin may be present but does not always appear in every patient.
- Papula: progression to muco-cutaneous lesion outbreak is rapid. Clinical manifestations of the lesion are papules (small, solid, inflammatory elevations of the skin that does not contain pus) or indurations of the skin.
- Vesicle: within the next 12 hours, lesions of maximal severity, known as cold sores or fever blisters appear on the vermillion border of the lip as multiple, fluid filled vesicles that tend to rupture rapidly and to become confluent into an ulcer in 72-96 hours. The lesion area and pain are generally maximum within 24 hours. This is also the stage at which a cold sore is most contagious.
   The first cold sores or fever blisters are named primary vesicles (or primary vesicular lesions). Sometimes, patients present secondary vesicles (or secondary vesicular lesions) which are other cold sores or fever blisters appearing after and around the first vesicles.
- Crust: then, the ulcer progresses to a crusting stage. In those areas where the cold sore lesion is not kept wet by moisture from the mouth the ulcer will become dry and scab over with a brownish crust. The formation of this scabbing is often accompanied by an itching or burning sensation. Often the scab will crack or break, which in turn produces bleeding. As time progresses so will the cold sore's healing.
- Erythema: during the healing erythema, a redness of the skin may be present but does not always appear in every patient.
- Healing/normal skin: the cold sore resolves itself fully, usually without scarring.

Herpes labialis typically resolves spontaneously within about 7-14 days. Although most of the episodes are mild, some may be severe or disfiguring, causing psychological distress and physical discomfort.

On average, 25% of all episodes do not progress beyond the papule stage. These are called "aborted" (or "abortive" or "non vesicular") episodes. About one-half of these do not progress beyond the prodromal stage.

Most patients undergo recurrent episodes throughout their life. That means that they have to manage several herpes labialis occurrences throughout their life. The infection is transmitted when the virus is present. The amount of virus is highest within the first eight hours of lesion development and diminishes as the lesions mature. Herpes labialis is spread through saliva or through the direct contact of skin or mucus membranes with lesions or oral secretions of an infected person. Risk of transmission often increases in day care settings due to the large numbers of children who are in close proximity to each other. Most of the transmission in these settings is believed to be asymptomatic. Transmission in households is believed to be from kissing, though it is good to avoid sharing cups, eating utensils, wash cloths, etc. when one has a visible sore.

In HIV patients, herpes labialis appears as chronic, hyperproliferative plaques different from the classic acute muco-cutaneous ulcerative lesions and may be related to resistant isolates.

Historically, the difficulty in treating herpes labialis has been attributed to the rapid development of lesions, natural history of viral infection and a strong secondary immunological response that limits lesion duration in untreated patients.

Viral replication is most active before prodromal symptoms or in the first 8 hours after their occurrence and a window of opportunity for antiviral agents may exist when adequate concentrations are used and treatment is initiated during the time that viral replication dominates temporarily the host immune response. Consequently, early high dose antiviral therapy is a logical treatment strategy and ultrashort, minute, treatments have currently been assessed, as described below

Currently, the only compounds recommended as the first line treatment of HSV diseases belong to the class of nucleoside analogues, competitive inhibitor of viral DNA polymerase. Acyclovir and penciclovir are current active principles used in the herpes labialis treatment. Acyclovir has been shown to be the most potent antiviral drug to treat herpes infection.

Several different formulations of acyclovir have been developed for the treatment and/or prevention of herpes infections: acyclovir 200 mg tablets, 5% acyclovir cream or acyclovir suspension for perfusion. Systemic bioavailability of the drug following administration via oral or topical routes is far from being optimal. The oral absorption of acyclovir is low and highly variable, leading to a poor bioavailability ranging from 15 to 30%. The percutaneous absorption of the 5% acyclovir cream is even poorer with limited transfer of the compound through the mucosa and the skin. The perfusion use is restricted to systemic infections occurring in immunocompromised patients.

In immunocompetent patients, a treatment regimen of acyclovir is 200 mg oral tablets, 5 times daily for 5 days. This treatment showed reduction of time for the loss of the crust (Raborn, J. Am. Dent. Assoc, 1987). However, acyclovir treatment is frequently prolonged due to its incomplete effect. Furthermore, studies have shown that this treatment had no effect on the duration of pain or the time to recovery (Raborn, Oral Surg Oral Med Oral Pathol Oral Radiol Endod, 1997).

Spruance et al. evaluated a regimen of 400 mg of oral acyclovir 5 times daily for 5 days (Spruance, J. Infect. Dis. 1990). Overall in this study, acyclovir reduces the pain and the time of lesion healing. However, a strong inconvenient of these two treatments is their incompliance for patient who have to take these tablets 5 times daily for 5 days.

In frequently recurrent herpes labialis (more than 5 occurrences per year), prophylactic treatment with acyclovir tablets is recommended (Rooney, Annals of Internal Medicine, 2004). Such treatment is, however, very inconvenient for patient since they have to take acyclovir tablets of 400 mg twice daily for 4 months. Currently, the only known prophylactic treatment of frequently recurrent herpes labialis is regular administration of antiviral tablet for a prolonged period of time (4 to 6 months).

The need for a drug with improved pharmacokinetic profile and clinical efficacy led to the development of valacyclovir (L-valine ester of acyclovir), a prodrug of acyclovir with increased bioavailability (absolute bioavailability of acyclovir following oral administration of valacyclovir: 54%). Valacyclovir is administrated via oral route (for instance Valtrex ® : containing 500 mg or 1000 mg of valacyclovir hydrochloride or Zelitrex®: containing 500 mg of valaciclovir Chlorhydrate) Likewise, famciclovir, a prodrug of pencyclovir - (diacetyl ester of 6-deoxy pencyclovir, the oral form of penciclovir with an absolute bioavailability of pencyclovir following oral administration of famciclovir of 77%) has been developed and registered for the early treatment of labial herpes. Recently, oral 4 g valacyclovir in 2 divided doses of valacyclovir (Valtrex ® 2 g twice daily for one day) (Spotswood L. Journal of Antimicrobial Chemotherapy, 2004) and 1.5 g famciclovir are administered within the first hour following prodromal symptoms of herpes labialis. These two treatments were efficient with high systemic doses administered early on for a short duration therapy. These treatments have been shown to induce a reduction of time to lesion healing versus placebo.

To summarize, valacyclovir or famciclovir-based treatments are oral treatments applied one or two times per day with high dosages (1.5g or 4g). These treatments show different drawbacks. Indeed, although reducing time to lesion healing, they do not prevent vesicular lesion and do not allow alleviation of rapid pain and other symptoms. Furthermore, side effect, and notably headaches (in about 10 % of treated patients) and/or nausea, are frequently reported, irrespective of the dosage and duration of treatment (Spruance, Antimicrob Agents Chemother 2003).

Topical treatments, such as ointments, are often the preferred option. Indeed, they allow an increased concentration of the active principle at the replication site and/or lesion sites. Presently, for patients developing orofacial herpes (1 to 5 episodes yearly), acyclovir or penciclovir creams or ointments are largely used due to their availability as over-the-counter or preparations without prescription in several countries. This also allows avoiding delays to obtain the mandatory prescriptions, which are required for oral acyclovir and valacyclovir. Although acyclovir in the cream or ointment vehicle had some effect in making cold sore lesions heal more quickly, it was not able to prevent cold sore lesions from arising, even when applied at the prodromal stage. The phenomenon of treatment-induced prevention of lesions is also referred to as "aborted lesions" and represents the holy grail of herpes simplex treatment (Spruance, Antimicrob. Agents Chemother. 2002). Such creams or ointments seem to be more active at the vesicular stage, because of easier transfer through the vesicular membrane or moisterizing properties. Several studies investigated the effects of acyclovir cream. However, none of them reported a decrease in the duration or severity of pain according to consensus (Opsteltel, Can Fam Physician 2008). Furthermore, the topical treatments demonstrated limited efficacy and required multiple applications over several days (Spruance , Herpes, 2002). Some studies have suggested that this limited efficacy is the result of inadequate penetration of the drug in the basal epidermis (Parry, J. Invest Dermatol, 1992). Finally, topical treatments are also incompliant for patients. For instance, patients have to apply topical penciclovir 1% cream every 2 hours during waking hours for 4 days or topical acyclovir 5% cream 5 times daily for 5 days.
International patent application WO2009/115510 relates to topical compositions comprising acyclovir, penciclovir and/or omaciclovir as the active principle. Conveniently, treatment with the composition of the invention is commenced as soon as the first sign of a herpes reoccurrence is detected, such as a tingling of the oral lesion or other manifestation of the prodromal stage. Advantageously the treatment results in an aborted lesion. It needs to be applied five times daily and continued for five days. Again, the recommended 5 daily applications for 5 days raises the issue of patient compliance. Furthermore, topical treatments have poor effects on pain and could generate local irritation. Document US2009/169511A1 discloses mucoadhesive buccal tablet suitable for daily or once-a-day dosing. Therefore, there is a need for another treatment and/or prevention for herpes labialis which also allows compliance for the patient, reduces global symptoms and/or is well tolerated.

Except for the oral (tablet or capsule) or the epidermal (cream or ointment) route, no other route of administration has been explored to improve bioavailability of acyclovir and/or increase the concentration at replication sites and/or lesion sites.

Therefore, it is an object of the present invention to fulfil said needs with a composition suitable to mucosal delivery of acyclovir and notably a labial and orofacial delivery.

Therefore it is also an object of the present invention to overcome the other deficiencies in the prior art of herpes labialis treatment.

### SUMMARY OF THE INVENTION

The present invention relates to a prolonged release mucoadhesive buccal tablet for use in the treatment and/or prevention of orofacial herpes wherein preferably a single dose is applied.

The invention is exemplified by the following numbered embodiments, which can be adapted according to the disclosure that follows.

A first embodiment (embodiment 1) of the invention is a method of preventing or ameliorating the severity of orofacial herpes in a patient infected with herpes simplex virus (HSV-1), comprising administering to the oral mucosa of said patient a mucoadhesive buccal tablet (*e*.*g*., by applying the mucoadhesive buccal tablet to the oral mucosa), said mucoadhesive buccal tablet comprising an effective amount of an acyclic guanosine antiviral agent and characterized by at least one, at least two, at least three, at least four, at least five or all six of the following properties:
(a) the mucoadhesive buccal tablet adheres to said oral mucosa for a period of at least 5 hours following administration;
(b) the mucoadhesive buccal tablet provides sustained release of said acyclic guanosine antiviral agent for a period of at least 20 hours following administration;
(c) the acyclic guanosine antiviral agent is acyclovir, and the mucoadhesive buccal tablet provides a maximum salivary acyclovir concentration ("Cmax") of at least 200,000 ng/ml;
(d) the acyclic guanosine antiviral agent is acyclovir, and the mucoadhesive buccal tablet provides a salivary acyclovir concentration of greater than 22.5 ng/ml for a period of at least 24 hours following administration;
(e) the acyclic guanosine antiviral agent is acyclovir, and the mucoadhesive buccal tablet provides time to maximum salivary acyclovir concentration ("Tmax") of 7 to 13 hours following administration; and
(f) the mucoadhesive buccal tablet comprises one, two, three, four or all five of the following components:
   (i) one or more diluents in a weight amount of 1% to 75%; 5% to 40%, or 10% to 20%;
   (ii) one or more solubilizing agents that do not facilitate absorption of said acyclic guanosine antiviral agent in a weight amount of 1% to 10%, 2% to 8%, or 4% to 6%;
   (iii) one or more binding agents in a weight amount of 0.1% to 5% or 0.1% to 2%;
   (iv) one or more bioadhesive polymers selected from the group of natural polymers wherein said natural polymers are polysaccharides or natural proteins from animal origin or vegetable origin, synthetic polymers, and mixtures thereof in a weigh amount of 5% to 80%, 10% to 50%, or 10% to 30%; and (v) one or more polymers that provide a sustained release of said acyclic guanosine antiviral agent in a weight amount of 5% to 80%, 10% to 50%, or 10% to 30%,
wherein (A) single dose of said acyclic guanosine antiviral agent is administered and/or (B) the patient is suffering from pre-vesicular symptoms of orofacial herpes.

Embodiment 2 of the invention is the method of embodiment 1, wherein a single dose of said acyclic guanosine antiviral agent is administered.

Embodiment 3 of the invention is the method of embodiment 1 or embodiment 2, wherein the patient is suffering from pre-vesicular symptoms of orofacial herpes, and wherein mucoadhesive buccal tablet is administered to the site of said non-pre-vesicular symptoms.

Embodiment 4 of the invention is the method of anyone of embodiments 1 to 3, wherein the patient is suffering from prodromal symptoms.

Embodiment 5 of the invention is the method of anyone of embodiments 1 to 4, wherein the mucoadhesive buccal tablet is administered within two hours, within 90 minutes, or within one hour of appearance of prodromal symptoms.

Embodiment 6 of the invention is the method of anyone of embodiments 1 to 5, wherein patient is suffering from erythemal or papular symptoms.

Embodiment 7 of the invention is the method of anyone of embodiments 1 to 6, wherein the patient is suffering from pre-vescicular symptoms of orofacial herpes and further comprising repeating said administration during a subsequent occurrence of pre-vesicular symptoms.

Embodiment 8 of the invention is the method of anyone of embodiments 1 to 7, wherein the mucoadhesive buccal tablet adheres to said oral mucosa for a period of at least 6 hours, at least 8 hours, at least 10 hours or at least 12 hours following administration.

Embodiment 9 of the invention is the method of anyone of embodiments 1 to 8, wherein the mucoadhesive buccal tablet provides sustained release of acyclovir for a period of at least 24 hours in at least 70% or, more preferably, in at least 80 %, of patients following administration.

Embodiment 10 of the invention is the method of anyone of embodiments 1 to 9, wherein the mucoadhesive buccal tablet provides a Cmax of at least 300,000 ng/ml, or at least 350,000 ng/ml, and optionally up to 400,000 ng/ml, up to 450,000 ng/ml or up to 500,000 ng/ml.

Embodiment 11 of the invention is the method of anyone of embodiments 1 to 10, wherein the mucoadhesive buccal tablet provides a salivary acyclovir concentration of greater than 22.5 ng/ml for a period of at least 30 hours following administration.

Embodiment 12 of the invention is the method of anyone of embodiments 1 to 11, wherein the mucoadhesive buccal tablet provides a Tmax of approximately 8 to 12 hours following administration.

Embodiment 13 of the invention is the method of anyone of embodiments 1 to 12, wherein the mucoadhesive buccal tablet provides a Tmax of approximately 8 hours or approximately 12 hours following administration.

Embodiment 14 of the invention is the method of anyone of embodiments 1 to 13, wherein the wherein said acyclic guanosine antiviral agent is acyclovir.

Embodiment 15 of the invention is the method of embodiment 14, wherein said mucoadhesive buccal tablet comprises acyclovir in an amount of 50 mg.

Embodiment 16 of the invention is the method of embodiment 15, wherein the mucoadhesive buccal tablet comprises acyclovir in an amount of 100 mg.

Embodiment 17 of the invention is the method of anyone of embodiments 1 to 16, wherein the mucoadhesive buccal tablet weighs 100 mg to 150 mg.

Embodiment 18 of the invention is the method of anyone of embodiments 1 to 17, wherein the mucoadhesive buccal tablet weighs 115 mg.

Embodiment 19 of the invention is the method of anyone of embodiments 1 to 18, wherein the mucoadhesive buccal tablet comprises:
(i) 5% to 40% of one or more diluents;
(ii) 2% to 8% by weight of one or more one or more solubilizing agents that do not facilitate absorption of said acyclic guanosine antiviral agent;
(iii) 0.1% to 2% by weight of one or more binding agents;
(iv) 10% to 50% by weight of one or more bioadhesive polymers selected from the group of natural polymers wherein said natural polymers are polysaccharides or natural proteins from animal origin or vegetable origin, synthetic polymers, and mixtures thereof; and
(v) 10% to 50% by weight of one or more polymers that provide a sustained release of said acyclic guanosine antiviral agent.

Embodiment 20 of the invention is the method of anyone of embodiments 1 to 19, wherein at least one of said one or more one or more solubilizing agents that do not facilitate absorption of acyclovir is an alkali metal alkylsulphate.

Embodiment 21 of the invention is the method of anyone of embodiments 1 to 20, wherein the alkali metal alkylsulphate is sodium sodium laurylsulphate.

Embodiment 22 of the invention is the method of embodiment 21, wherein the mucoadhesive buccal tablet comprises sodium laurylsulphate in an amount of at least 2% by weight or at least 4% by weight.

Embodiment 23 of the invention is the method of embodiment 22, wherein the mucoadhesive buccal tablet comprises sodium laurylsulphate in an amount of approximately 4.5% by weight.

Embodiment 24 of the invention is the method of anyone of embodiments 1 to 23, wherein at least one of said one or more diluents is microcrystalline cellulose.

Embodiment 25 of the invention is the method of anyone of embodiments 1 to 24, wherein at least one of said one or more binding agents is povidone.

Embodiment 26 of the invention is the method of anyone of embodiments 1 to 25, wherein at least one of said one or more polymers that provide a sustained release of acyclovir is hypromellose.

Embodiment 27 of the invention is the method of anyone of embodiments 1 to 26, wherein at least one of said one or more bioadhesive polymers is milk protein concentrate.

Embodiment 28 of the invention is the method of anyone of embodiments 1 to 27, wherein the mucoadhesive buccal tablet is made by a method comprising:
(a) mixing said acyclovir with an alkali metal alkylsulfates and a diluent to form a mixture;
(b) wetting said mixture produced in (a) in the presence of a binding agent;
(c) drying and calibrating said mixture produced in (b);
(d) granulating said mixture produced in (c) to form primary granules;
(e) blending said primary granules with a bioadhesive polymers, a sustained release polymer and a compression agent; and
(f) compressing the blended mixture obtained in (e).

In another aspect the present invention relates to a prolonged release mucoadhesive buccal tablet comprising at least one acyclic guanosine antiviral agent, 1 to 75% by weight of a diluent, and 1 to 10% by weight of an alkali metal alkylsulfate, 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5% to 80% by weight of at least one polymer that provides a sustained release of acyclic guanosine antiviral agent.

The prolonged release mucoadhesive buccal tablet can be used in a long term efficient treatment and/or prevention for use in the reduction of the duration of abortive episodes, for use in reduction of the duration of orofacial herpes episodes, for use in reduction of the occurrence of secondary vesicular lesions, for the treatment and/or prevention of orofacial herpes without significant side effect due to said composition, for use in reduction of the time of healing of primary or secondary vesicular lesions, for use in reduction of global symptoms intensity of orofacial herpes,for use in reduction of salivary viral load and thus local viral spread, intra-individual and/or inter-individual viral transmission, and/or for use at the prodromal stage of herpes labialis.

In another aspect the present invention relates to a prolonged release mucoadhesive buccal tablet comprising at least one acyclic guanosine antiviral agent, 1 to 75% by weight of a diluent, and 1 to 10% by weight of an alkali metal alkylsulfate, 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5% to 80% by weight of at least one polymer that provides a sustained release of the at least one acyclic guanosine antiviral agent.

In yet another aspect the present invention provides a prolonged release mucoadhesive buccal tablet comprising at least one acyclic guanosine antiviral agent, 1 to 75% by weight of a diluent, and 1 to 10% by weight of an alkali metal alkylsulfate, 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5% to 80% by weight of at least one polymer that provides a sustained release of the active principle for use in the treatment of orofacial herpes.

Other aspects of the mucoadhesive buccal tablet used in accordance with the present invention can be found in international patent application publication WO2007/110778, which is incorporated herein by reference in its entirety.

### BRIEF DESCRIPTION OF THE DRAWINGS

**FIG. 1** is a schematic representation of the evolution of herpes labialis pathology
**FIG. 2** is a schematic representation of the process to produce the prolonged release mucoadhesive buccal tablet of the present invention.
**FIG. 3** Represents the time to healing in mITT population
**FIG. 4** Represents the proportion of patients without recurrence of herpes labialis.
FIG. 5 is a graph showing the concentration acyclovir in the plasma over time in hours using the bioadhesive slow release carrier of the present invention compared with that of an oral acyclovir tablet.
FIG. 6 is a graph showing the concentration of acyclovir in the saliva over time in hours using the bioadhesive slow release carrier of the present invention compared with that of an oral acyclovir tablet.
FIG. 7 is a graph showing the concentration of acyclovir on the lips over time in hours using the bioadhesive slow release carrier of the present invention compared with that of an oral acyclovir tablet.

### DESCRIPTION OF THE PREFERRED EMBODIMENTS OF THE INVENTION

The term "muco-cutaneous" means a zone of skin comprising both mucosa and cutaneous skin. These mostly occur near the orifices, at the lips for instance.
The term "buccal" means a zone lying in the mouth.
The term "treatment" means alleviating, inhibiting the progress of, or curing orofacial herpes, or one or more symptoms of orofacial herpes.
The term "symptoms" means itching, tingling, pain, burning, vesicular lesion, discomfort and tenderness.
The term "prevention" means reducing the frequency of recurrence of orofacial herpes.

The term "long term prevention" means reducing the frequency of recurrence of Orofacial herpes within at least 9 months after the last occurrence.
Similarly, throughout the text, the expression "prolonged release"; "slow release" or "sustained release" are used interchangeably and mean that the active principle is released immediately after 30 minutes and then over a prolonged period of time of at least 15 hours or at least 18 hours or at least 20 hours or at least 24 hours and up to 36 hours.
The term "occurrence" means the beginning of an episode of orofacial herpes.
The term "recurrence" means the occurrence of new episode of orofacial herpes, after the healing of all lesions of the initial episode of orofacial herpes
The expression "frequently recurrent orofacial herpes" means more than 5 occurrences per year.
The expression "severe orofacial herpes" means persistent and/or spread lesions in particular in the immunocompromized patient.

"Orofacial herpes" means an herpes with facial or cutaneous or mucosal lesions or stomatitis.
The expression "time to recurrence" means the time from the healing of all lesions of the initial episode of orofacial herpes to the occurrence of new lesions.
The expression "primary vesicular lesion" is the first developed lesion which should be located on the lip and should not extend more than 1cm outside the lip. Pure intra-oral lesions are not considered as primary lesions.
The expression "aborted lesions" means herpetic lesions preceded by prodromal symptoms that do not progress beyond the papule stage.

The expression "secondary lesions" means the lesions developed in addition to and/or in 1 or more days after the primary lesion and that are located at least 1cm from the primary lesion.
The expression "duration of episode" means the time from the beginning of prodromal symptoms to the healing of primary and secondary lesions.
The expression "duration of abortive episode" means the time from the beginning of prodromal symptoms to the healing of the papula lesions.
The expression "time to cessation of symptoms" means the time from the treatment initiation to the cessation of all symptoms: pain, burning, itching, tingling, vesicular lesion, tenderness and discomfort.
The expression "time to healing of aborted primary lesions (TTH)" means the time from the treatment initiation to the healing of primary lesion or cessation of symptoms, whichever comes last.
The expression "time to healing of intra-oral/mucosal secondary lesions" means the time from the treatment initiation to healing of intra-oral/mucosal non primary lesions.
The term "healing" means the loss of crust. Erythema may be present following the loss of crust.
The expression "time to healing" means the time from the treatment initiation (date and hour recorded) to the healing.
The expression "ITT Population" means all randomized patients who took at least one dose of the study medication.
The expression "mITT population" means all randomized patients who took at least one dose of the study medication and who reached the vesicular stage.
The expression "PP population" means all patients of the mITT population who did not have a major protocol deviation.

The prolonged release mucoadhesive buccal tablets of the invention are described in the patent application US20090169511, incorporated herein by reference.
The invention relates to the use of the prolonged release mucoadhesive buccal tablets for mucosal, notably labial, slow release of acyclic guanosine antiviral agents for the treatment of orofacial herpes. Said prolonged release mucoadhesive buccal tablets have many advantages as described below.
The main general advantage of the prolonged release mucoadhesive buccal tablets of the invention is to allow a rapid release (30 minutes after application) of acyclic guanosine antiviral agents in saliva and for a long duration (more than 24 hours). As demonstrated below, this advantageous kinetic release leads to an early (in the first days) and prolonged (within at least 9 months) efficient treatment and/or prevention of orofacial herpes. Furthermore the prolonged release mucoadhesive buccal tablets of the invention are used at a single dose and surprisingly afford several kinds of relief for patients undergoing orofacial herpes. This single dose notably allows preventing cold sore lesions from arising. In other words, the mucoadhesive buccal tablets of the invention increases the number of "aborted lesions" in treated patients. Furthermore, said single dose allows a much better compliance for patients, compared to the treatments of the state of the art and notably to the current topical treatments, needing application five times daily and continued for five (or more) days.
Another embodiment of the invention is to provide prolonged release mucoadhesive buccal tablets for the treatment of orofacial herpes with a low dose of acyclovir. This low dose is comprised between 10 to 200 mg per tablet and 50 mg is preferred. Hence the mucoadhesive buccal tablets of the invention allow an efficient treatment and/or prevention with a low active principle dosage. It is surprising in comparison to the current treatments where high doses of active principle are preferred. An unexpected efficacy with respect to aborted lesions has been shown. Indeed, the mucoadhesive buccal tablets of the invention substantially reduce the number of patients who progress beyond the papule stage. In other words the number of patients that enter the vesicular lesion stage is substantially reduced. This result is all the more noteworthy since it is obtained with a single dose. This result is especially marked when applied on patients undergoing the prodromal symptom. Hence, an object of the invention is to provide prolonged release mucoadhesive buccal tablets for the treatment of orofacial herpes, especially at the prodromal stage. As orofacial herpes is most contagious at the vesicular stage, it is another advantage of the prolonged release mucoadhesive buccal tablets of the invention to reduce the risk of transmission of the virus by limiting the occurrence of vesicular lesions.
It has also been demonstrated that the mucoadhesive buccal tablets of the invention reduce the duration of abortive episodes. Thus another aspect of the invention relates to the use of prolonged release mucoadhesive buccal tablets on patients undergoing the prodromal symptom to decrease the duration of abortive episodes.

A further aspect of the invention is to provide prolonged release mucoadhesive buccal tablets to reduce the duration of orofacial herpes episodes. Indeed, a remarkable decrease of the global duration of episodes has been demonstrated. Since this effect has been observed on the global duration of episodes, it is expected that the prolonged release mucoadhesive buccal tablets of the invention can be therapeutically efficient even if applied after the prodromal stage.

Another aspect of the invention relates to prolonged release mucoadhesive buccal tablets for reducing the duration or severity of symptoms (itching, tingling, pain, burning, discomfort, and/or tenderness) induced by herpes labialis. Surprisingly, the prolonged release mucoadhesive buccal tablets show an early efficacy. Indeed, significant reduction of global symptom intensity begins at day 3 (thus during the abortive episode, as defined in Figure 1) and is the most significant at day 5.

More precisely, studies conducted herein have demonstrated that the time of cessation of symptoms is about 0.4 days shorter with the prolonged release mucoadhesive buccal tablets of the invention than with placebo.

A further embodiment of the invention relates to prolonged release mucoadhesive buccal tablets for the treatment of orofacial herpes by reducing the occurrence of secondary vesicular lesions. Indeed, it has been showed that the prolonged release mucoadhesive buccal tablets reduces the occurrence of secondary lesions by nearly 50% as compared to placebo.

Another embodiment of the invention is to provide prolonged release mucoadhesive buccal tablets for the treatment of orofacial herpes without adverse side effects due to acyclovir. Indeed prolonged release mucoadhesive buccal tablets of the invention are extremely well tolerated and present very limited systemic effects (notably diarrhea, headache) and, contrary to the current topical treatments, do not induce local irritation.

A further aspect of the invention is to provide prolonged release mucoadhesive buccal tablets for the treatment of orofacial herpes by significantly reducing the time of healing of primary vesicular lesion.

More precisely, studies conducted herein have demonstrated that the time of healing is about 0,4 days less than placebo.

Another aspect of the invention relates to prolonged release mucoadhesive buccal tablets for the treatment of orofacial herpes by reducing the recurrence of abortive episodes or episodes. Indeed, it has been shown herein for the first time prevention of the recurrence of vesicular lesions. Prolonged release mucoadhesive buccal tablets of the invention are thus particularly suitable for the treatment of recurrent orofacial herpes or severe orofacial herpes.

Another object of invention is to provide prolonged release mucoadhesive buccal tablets for an early or short term efficient treatment and/or prevention of abortive episodes or episodes.

Another object of invention is to provide prolonged release mucoadhesive buccal tablets for the treatment of frequently recurrent and/or severe orofacial herpes especially for immunocompromized patients.

The prolonged release mucoadhesive buccal tablets of the invention are also suitable for the treatment of not only cutaneous lesions but also mucosally, spread, lesions. It is another object of the invention to provide the use of prolonged release mucoadhesive buccal tablets for mucosal delivery of acyclovir allowing maintaining efficacious concentrations of acyclic guanosine antiviral agents in the viral reservoir, decreasing local viral spread and thus intra-individual and inter-individual viral transmission. Indeed, besides early and prolonged efficient concentrations of acyclic guanosine antiviral agents in the saliva, the prolonged release mucoadhesive buccal tablets of the invention allow early and prolonged efficient concentrations of acyclic guanosine antiviral agents in the mucous, epidermis and/or trigeminal ganglions.

Another embodiment of the invention is to provide prolonged release mucoadhesive buccal tablets for the treatment of orofacial herpes with a low dose of acyclovir. Said low dose is comprised between 10 to 200 mg per tablet, while 50 mg is preferred.

It is another aspect of the invention to provide the use of prolonged release mucoadhesive buccal tablets for mucosal delivery of acyclovir allowing an early and sustained release of acyclovir at the infection site to exert a "continuous antiviral pressure" against HSV-1.
Another aspect of the invention is to provide the use of prolonged release mucoadhesive buccal tablets for mucosal delivery of acyclovir attached to the buccal cavity, retained for a longer period of time and removed at any time, thus allowing flexibility of use.
It is another aspect of the invention to provide the use of prolonged release mucoadhesive buccal tablets for mucosal delivery of acyclovir which can be applied at different sites in the oral cavity, preferably at the gum.
The prolonged release mucoadhesive buccal tablets of the invention are described in patent application US20090169511, incorporated herein by reference.
These prolonged release mucoadhesive buccal tablets at least one acyclic guanosine antiviral agent, 1 to 75% by weight of a diluent, and 1 to 10% by weight of an alkali metal alkylsulfate, 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5% to 80% by weight of at least one polymer that provides a sustained release of the acyclic guanosine antiviral agents.
Examples of diluents include microcrystalline cellulose, silicified microcrystalline cellulose, hydroxymethylcellulose, dicalcium phosphate, calcium carbonate, calcium sulfate, magnesium carbonate, tricalcium phosphate, lactose, starck and the like. The diluent is present in an amount between 1% to 75%, 5% to 40 %, or 10% to 20% by weight in the bioadhesive slow release carrier.
An alkali metal alkylsulfate is also a component of the prolonged release mucoadhesive buccal tablets of the present invention. This alkali metal sulfate improves the granulation of the active principle acting as a solubilization agent. The alkali metal alkylsulfate that can be used in the formulation includes magnesium lauryl sulfate, potassium lauryl sulfate, sodium laurylsulfate and diethylsulphosuccinate. Generally it is present in the prolonged release mucoadhesive buccal tablets at a concentration of between 1 to 10% by weight, 2% to 4% by weight, 2% to 6% by weight, 2% to 8% by weight, or 2% to 10% by weight.
The binders used in the present invention can be selected from carboxy vinyl polymer, methycellulose, hydroxyethylcellulose, hydroxypropyl cellulose, gumarabic, starch, hydroxypropylmethylcellulose, polyvinylpyrrolidone, polyethylene glycol and the like. The binders are present in the amount of 0.1% to 5% or 0.1% to 2% by weight in the bioadhesive slow release carrier.
The bioadhesive polymers are selected from the group of:
- natural polymers :
   o Polysaccharides: chitosan, alginate, carboxymethyl cellulose, hydroxypropyl methyl cellulose (also called hypromellose), hydroxyethyl cellulose, hydroxypropyl cellulose, cyclodextrin, sodium hyaluronate, xanthum gum,
   o Natural proteins: from animal origin or vegetable origin, natural milk proteins, natural pea proteins, natural soy proteins, natural potato proteins, natural wheat proteins, gliadin proteins,
- synthetic polymers: carbomer, polyvinylalcohol, acrylic polymers.
The bioadhesive polymers are present in the prolonged release mucoadhesive buccal tablets prolonged release mucoadhesive buccal tablets at a concentration of 5% to 80% by weight. They can also be present in an amount of 10% to 30% by weight, 10% to 40% by weight or 10% to 50% by weight. Regarding the natural milk proteins, these are described in EP 0 542 824. They can be obtained from pasteurized raw milk and include total milk proteins, casein protein concentrates and whey protein concentrates. The total milk proteins are recovered from skimmed milk after ultrafiltration. The casein protein products are obtained by insolubilizing the casein in milk at its isoelectric point, and further washing and drying the casein. The whey protein concentrates are obtained after coagulating cheese with enzymes and separating the yellow-green liquid residue out, which residue is whey. The whey is then further concentrated by ultrafiltration, ion exchange chromatography or thermal precipitation. Regarding the vegetable proteins, they can be obtained from pea, soy, potato, wheat or gliadin. The method for producing pea protein is described in WO 2007/017571.
The sustained release polymers that can be used in the prolonged release mucoadhesive buccal tablets include hydrophilic polymers including polysaccharides such as cellulose ethers, xanthum gum, scleroglucan, locust bean gum, gum Arabic, gum tragacanth, carob, alginic acid, alginates, carrageenates, agar-agar and guar gum either alone or in mixtures thereof. Other polymers that can be used in the present invention include cellulose based polymers such as hypromellose (also named hydroxypropyl methyl cellulose), cellulose acetate, cellulose esters, cellobiose, cellulose resins alone or in mixtures thereof. The sustained release polymers are present in a concentration of 5% to 80% by weight. They can also be present in an amount of 10% to 30% by weight, 10% to 40% by weight or 10% to 50% by weight.
In another embodiment the present invention relates to a mucosal bioadhesive slow release carrier comprising 10 to 200 mg of acyclovir, 1 to 75% by weight of a diluent of microcrystalline cellulose and 2 to 10% by weight of sodium lauryl sulphate and further comprising 0.1 to 5% by weight of a polyvinylpyrrolidine and 10 to 40% by weight of at least one bioadhesive polymer selected from the group of natural milk proteins and mixtures thereof and 10% to 40% by weight of hypromellose.
Magnesium stearate between 0.1 to 5 % and colloidal silica between 0.1 to 1% can be added to the mucosal bioadhesive slow release carrier to facilitate the process of preparation. The at least one acyclic guanosine antiviral agent is preferably acyclovir.
The mucosal bioadhesive slow release carrier used in the present invention permits the immediate local liberation of the active principle, as well as the prolonged liberation of the active principle and then provides an early and prolonged efficacy.
The preferred embodiment of the invention is a prolonged release mucoadhesive buccal tablets comprising 50 mg of acyclovir, 15% by weight microcrystalline cellulose, 4.5% by weight of sodium lauryl and further comprising 0.4 % by weight of polyvinylpyrrolidine, 20% by weight of milk concentrate protein, 15% by weight of hypromellose, 0.5% by weight of magnesium stearate and 0.4 % by weight of of colloidal silica.
A method for preparing said prolonged release mucoadhesive buccal tablets comprises:
a) granulating a mixture of at least one acyclic guanosine antiviral agent with an alkali metal alkylsulfate, a diluent and a binding agent;
b) blending said granulated mixture with at least one bioadhesive polymer, at least one sustained release polymer and at least one compression agent; and
c) compressing the blended mixture obtained in b).
In yet another aspect the present invention provides a method for delivering at least one acyclic guanosine antiviral agent to a mammal, said method comprising mucosally administering to a mammal in need of said at least one acyclic guanosine antiviral agent, a bioadhesive slow release carrier comprising at least one acyclic guanosine antiviral agent, 1 to 75% by weight of a diluent and 1 to 10% by weight of an alkali metal alkylsulfate 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5 to 80% by weight of at least one polymer that provides a sustained release of the at least one acyclic guanosine antiviral agent.
A number of embodiments of the invention have been described. Nevertheless, it will be understood that various modifications may be made without departing from the spirit and scope of the invention.

### EXAMPLES

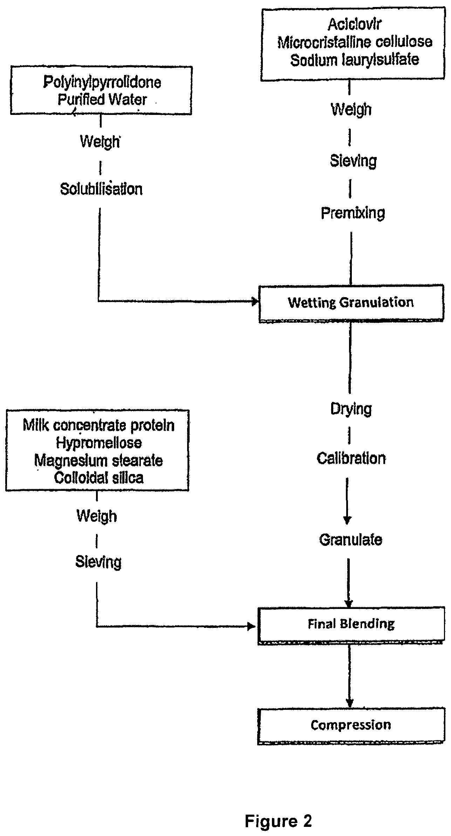

### EXAMPLE 1 - PREPARATION OF A PROLONGED RELEASE MUCOADHESIVE BUCCAL TABLET CONTAINING 50MG OF ACYCLOVIR (FIGURE 2)

50 mg of acyclovir, 15% by weight microcrystalline cellulose and 4,5% by weight of sodium lauryl sulfate were weighed and sieved with a 0.7 to 2 mm sieve before premixing in a blender to provide the "initial mix."

At the same time, 0.4 % by weight polyvinylpyrrolidone was dissolved in purified water: The resulting solution was added to the initial mix and further stirred. The wetted mixture was then granulated using a pharmaceutical mixer or granulator such as a planetary mixer or high sear mixer and dried and then calibrated to 0.5 to 2 mm. The resulting pellets formed the "primary granules."

20% milk protein concentrate, 15% hypromellose, 0.5% to 1% magnesium stearate and 0.4 % of colloidal silica were weighed and sieved using a 0.5 to 2 mm sieve. These ingredients were then added to the primary granulation to form the "final blending" mixture. The final blending mixture was then compressed using a tablet press such as a rotative press to produce the compressed carriers according to the invention.

The size of the tablets was about 8 to 10 mm in diameter. The dimension was chosen to be comfortable in the canine fossa.

The quantitative and qualitative composition of a preferred tablet of the invention is indicated in the following table.

| **Compound** | **Function** | **Quantity per tablet (mg)** | **Quantity per tablet (% w/w)** |
|---|---|---|---|
| Acyclovir | active principle | 50.000 | 43.48 |
| Microcristalline cellulose | tablet diluent | 18.040 | 15.19 |
| Povidone | tablet binder | 0.500 | 0.43 |
| Sodium Laurylsulfate | wetting agent | 5.175 | 4.5 |
| Hypromellose | sustained release agent | 17.250 | 15 |
| Milk Protein Concentrate | mucoadhesive agent | 23.000 | 20 |
| Magnesium Stearate | lubricant | 0.575 | 0.5 |
| Colloidal Silica | glidant | 0.460 | 0.4 |
| Purified water* | Wetting agent | qs (qs = quantum sufficit) | qs (qs = quantum sufficit) |
| | Total | 115 | 100 |
| * Removed during processing. Not present in the finish product | | | |

### EXAMPLE 2 - INVESTIGATIONAL PLAN

A randomised, double-blind, single dose multicenter study comparing a single dose treatment of "acyclovir Lauriad® 50mg", the prolonged release mucoadhesive buccal tablets of Example 1 (hereinafter referred to as AMBT 50 mg), versus matching placebo (randomisation in a 1:1 ratio) in immunocompetent patients suffering from recurrent orofacial herpes was carried out.

The study was carried out at multiple centers that treat patients with orofacial herpes. Approximately 1730 patients who meet the inclusion/exclusion criteria, were randomised according to a 1:1 ratio to receive either a single dose of AMBT 50mg, or matching placebo. Only those patients having an episode of orofacial herpes in the 6 months following randomisation had applied the tablets. It was calculated that 780 patients (390/group) had to be included to adequately compare the efficacy of AMBT 50 mg to placebo; 1170 patients were therefore randomised and not treated.

The study duration for each patient included a screening period of 10 days maximum before randomisation. The patient then waited for a new orofacial herpes episode to occur (up to a maximum of 6 months). If the patient did not experience an episode of orofacial herpes within 6 months after randomisation he/she was excluded from the study. As soon as the patient experienced prodromal symptoms, he/she self-initiated his/her treatment. The patient was under evaluation up to Day 14, or up to the healing of primary lesions, whichever came first. For those patients in which the treatment was initiated, the maximum patient participation duration was 204 days (including the 6 months period). In some selected centers, patients were required to record the number of new episodes and the time to their recurrence for a period of 9 months.

For patients who were not treated because of the absence of recurrence of orofacial herpes in the 6 months following randomisation, the patient participation was 190 days.

### Inclusion criteria

Patients were randomised provided that they satisfied the following criteria:
- Male or female;
- Age > 18 years;
- History of recurrent herpes labialis lesions where:
   - Recurrence was defined as at least 4 episodes in the preceding 12 months;
   - Herpes labialis lesions were caracterised by their localisation on the cutaneous and/or mucosal surfaces of the lips;
- At least 50% of previous episodes produced classical lesions progressing to the vesicular stage (i.e., episodes that progressed through macula, papule, vesicle, crust and healing);
- Prodromal symptoms (itching, tingling, pain etc.) preceded herpes labialis lesions in at least 50% of the recurrent episodes;
- Good general health (ECOG < 2), immunocompetent;
- Signed and dated written informed consent;
- Women of childbearing potential were included provided that they use an highly effective contraception method: hormonal contraception, implantable contraceptive, injectable contraceptive double-barrier method (diaphragm with spermicide, codom with spermicide) or intrauterine device, at least 1 month prior to study start and throughout the entire duration of the study;
- Subjects had to agree to abstain from any mechanical disruption of the prodromal area or lesion (i.e. scrubbing, lancing, shaving the area, rubbing with alcohol,...).

### Endpoints

To demonstrate the efficacy of a single dose of AMBT 50mg versus a single dose of matching placebo of herpes labialis the endpoints were:
- The evolution of prodromal symptoms to aborted lesions;
- The healing of primary lesions;
- The healing of non primary lesions;
- The duration of episode;
- The duration of symptoms;
- The intensity of global symptoms;
- The healing of aborted lesions;
- The healing of intra-oral and mucosal lesions;
- The incidence of and time to recurrence during 9 months following treatment (ancillary study in selected centers);
- The comparison of the local tolerability and general safety of AMBT 50mg to those of placebo;
- The evaluation of the concentration of acyclovir in saliva (ancillary study in selected centres) and the assessment of its relationship with viral load in saliva and efficacy criteria;
- To evaluate the adhesion time of AMBT 50 mg, the incidence of detachment and / or swallow and the number of tablets replaced.

### Adverse events

An adverse event was considered as drug related if a causal relationship could not be excluded. In case of missing causal relationship the adverse event was considered drug related.

Treatment emergent adverse events (TEAE) was evaluated. A TEAE is defined as:
- An adverse event that occurs after starting the study treatment and that was not present before administration of the study treatment;
- An event that was present before study treatment but worsened in intensity or frequency after administration of the study treatment.

The treatment emergent period went from treatment administration to the end of the study.

A patient listing of all reported adverse events was prepared.

### Haematology and biochemistry parameters

A descriptive analysis by treatment group was performed. Abnormal values considered as clinically significant were identified.

### Local tolerability

A descriptive analysis by treatment group was performed.

### Compliance

A detailed description was made regarding compliance and the duration of the two treatments. Comparison of compliance and treatment duration between the two groups was made.

### Results

**Patient population:**

| **Patients** | **AMBT 50 mg** | **Placebo** | **Total** |
|---|---|---|---|
| Included | | | 1727 |
| Treated | 378 (100%) | 397 (100%) | 775 (44.9%) |
| ITT (patients who took at least one dose of AMBT 50mg) | 376 (99.5%) | 395 (99.5%) | 771 (99.5%) |
| mITT (Patients who reached the vesicular stage) | 242 (64.0%) | 279 (70.3%) | 521 (67.3%) |
| Patient with borted episodes | 130 (34.9%) | 109 (28.1 %) | 239 (31.4%) |

The skilled person is able to carry out known statistical analysis method to obtain the following results:

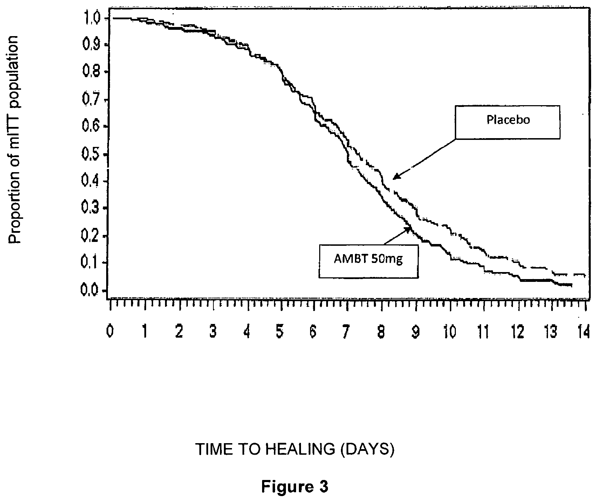

The time to healing of primary vesicular was significantly reduced compared to the placebo (cf. figure 3)

| . | AMBT 50 mg | Placebo |
|---|---|---|
| Patients | 242 | 279 |
| Time to healing | 7.03 ± 0.18 | 7.57 ± 0.19 |

AMBT 50 mg significantly preventeed the occurrence of vesicular lesions and significantly reduced the duration of non vesicular episodes

| | AMBT 50 mg | Placebo |
|---|---|---|
| Patients | 376 | 395 |
| Patient (%) with aborted episodes | 130 (34.9%) | 109 (28.1 %) |
| Duration (days) | 2.41 | 2.49 |

AMBT 50 mg significantly reduced the duration of episodes (aborted episodes and vesicular episodes)

| | AMBT 50 mg | Placebo |
|---|---|---|
| Patients | 376 | 395 |
| Duration (days) | 5.50 ± 0.17 | 6.11 ± 0.18 |

AMBT 50 mg significantly reduced the duration of orofacial herpes symptoms.

| | AMBT 50 mg | Placebo |
|---|---|---|
| Patients | 376 | 355 |
| Duration (days) | 4.07 ± 0.16 | 4.54 ± 0.17 |

Reduction of global symptoms intensity begun at day 3 and was the most significant at day 5. AMBT 50 mg rapidly decreased pain associated with herpes. Global symptoms intensity was self recorded by patients with a Visual Analog Scale (VAS), well known by the skilled person. The unit is the millimeter (mm). The more are the mm, the more are pain associated with herpes

| | AMBT 50 mg | Placebo |
|---|---|---|
| Patients | 378 | 397 |
| Day 1 (mm) | 30.5 | 31.1 |
| Day 3 (mm) | 21.2 | 22.9 |
| Day 5 (mm) | 12.7 | 17.3 |
| Day 7 (mm) | 8.2 | 10.7 |
| Day 14 (mm) | 1.0 | 0.9 |

AMBT 50 mg significantly reduced the occurrence and time to healing of secondary lesions

| | AMBT 50 mg | Placebo |
|---|---|---|
| Patients | 39 (10.3%) | 58 (14.6%) |
| Duration (days) | 8.70 ± 0.57 | 10.11 ± 0.48 |

AMBT 50 mg was extremely well tolerated, without difference compared to the placebo with respect to side effects of treatment (diarrhea, headache and/or site irritation) and had no effect on haematology and biochemistry parameters.

| | AMBT 50 mg | Placebo |
|---|---|---|
| Patients | 378 | 397 |
| Patients who complained of side effects of treatment | 27(7.1%) | 31 (7.8%) |

| | AMBT 50 mg | Placebo |
|---|---|---|
| Patients | 376 | 395 |
| Patients satisfied by the treatment | 297 (81.8%) | 275 (72.4%) |

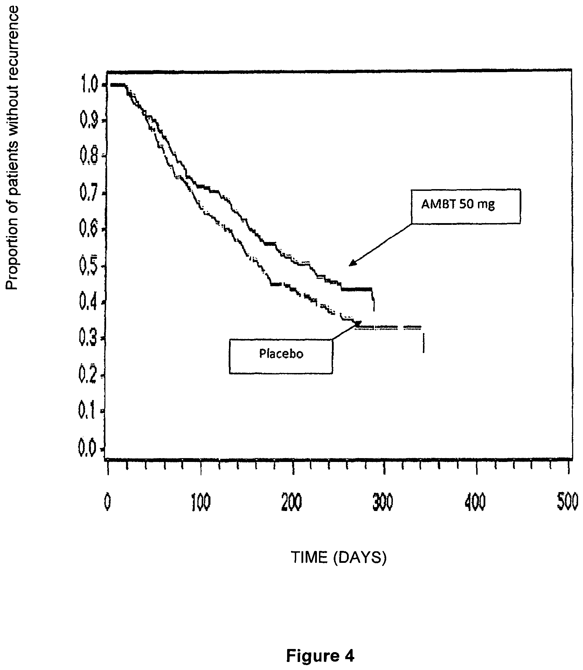

AMBT 50 mg allowed a remarkable difference in term of recurrence of aborted episodes or episodes. Within a follow-up of 9 months, a single dose of AMBT 50 mg reduced the number of patients in whom orofacial herpes recurred and in those patient with recurrence, that said new episode appeared at least 1 month later compared to the placebo (cf. figure 4).

| | AMBT 50 mg | Placebo |
|---|---|---|
| Patients | 114 (45.1 %) | 144 (55.0%) |
| Median (days) | 217 | 161 |

The effects were more marked when patients applied AMBT 50mg within 1 hour following the first prodromal symptoms.

| | AMBT 50 mg | Placebo |
|---|---|---|
| Median (days) | 248 | 165 |

Furthermore, AMBT 50 mg significantly reduced the risk of transmission of the virus by limiting the occurrence of vesicular lesions.

To summarize, the complete evaluation of the study is demonstrative regarding the primary endpoint Time to healing of primary vesicular lesion with a p= 0.043, secondary endpoints (less patients presenting vesicular lesions in the course of the infection and shorter global duration of the episode , decreased intensity of symptoms) were also significant. The tolerance was remarkable. The long term follow up showed a clear trend to prevent recurrence of episodes at 9 months.

A Phase I pharmacokinetic/pharmacodynamic (PK/PD), single-center, open, randomized, 3-period cross-over study to compare the pharmacokinetic (PK) parameters and the tolerability of a single dose of acyclovir Lauriad® 50mg mucoadhesive buccal tablet (MBT) and 100mg MBT in plasma, saliva and labial mucosa to those of acyclovir 200 mg regular tablet (Zovirax®). The 3 treatment periods were separated by a washout period of 7 days. All three treatments were administered in the morning as single dose: acyclovir Lauriad® 50 mg or 100 mg MBT was applied to the upper gum just above the incisor tooth and was to remain in the oral cavity until complete erosion or detachment, and acyclovir oral tablet 200 mg was administered with a glass of water. Concentrations of acyclovir in plasma, saliva and labial samples were measured using a validated high-performance liquid chromatographic (HPLC) method (Lower limit of quantification (LOQ): 10ng/mL).

Examples 3 to 7 below describe various aspects of the phase I study.

### EXAMPLE 3 - PHARMACOKINETICS OF THE ACYCLOVIR BIOADHESIVE SLOW RELEASE CARRIER

The main goal of this pharmacokinetic study was to evaluate the systemic passage of the acyclovir following the application of the bioadhesive slow release carrier at the level of the canine fossa (upper gingiva) in healthy volunteers. Additional goals were to evaluate the loco-regional concentrations of acyclovir in the saliva, which represents a virus reservoir site, and at the labial level, which constitutes the expression site for an herpes simplex 1 infection.

In order to evaluate the absorption level of acyclovir through the new mode of administration of the present invention, data was obtained with the bioadhesive slow release carrier of the present invention and compared to oral administration of 200 mg acyclovir tablets. Further, to evaluate the therapeutic potential of the new bioadhesive slow release carriers of the present invention, plasma and locoregional concentrations were compared to the minimal inhibitory concentration (MIC) of acyclovir towards HSV-1 virus, which is 22.5 ng/ml.

The study was undertaken using 12 healthy volunteers and was a monocentric, randomized, cross-over and open evaluation. Two acyclovir bioadhesive slow release carriers synthesized according to Example 1 were tested, containing either 50 mg or 100 mg of acyclovir.

Plasma, salivary and labial (lip) samples were taken prior to the administration of the treatment, and then regularly at 24 hours, 36 hours and 48 hours after administration. Labial sampling was accomplished by utilizing a stripping method; i.e., an adhesive disk was used to collect the superficial cell layers of the lip. To avoid lip contamination with saliva, labial sampling was performed prior to saliva sampling after the lips were carefully wiped.

The acyclovir was then extracted and measured by HPLC. The quantification limit was set at 10 ng/ml for plasma and saliva samples, and at 6.5 ng/cm2 for labial samples.

### EXAMPLE 4 - EVALUATION OF THE ACYCLOVIR SYSTEMIC TRANSFER

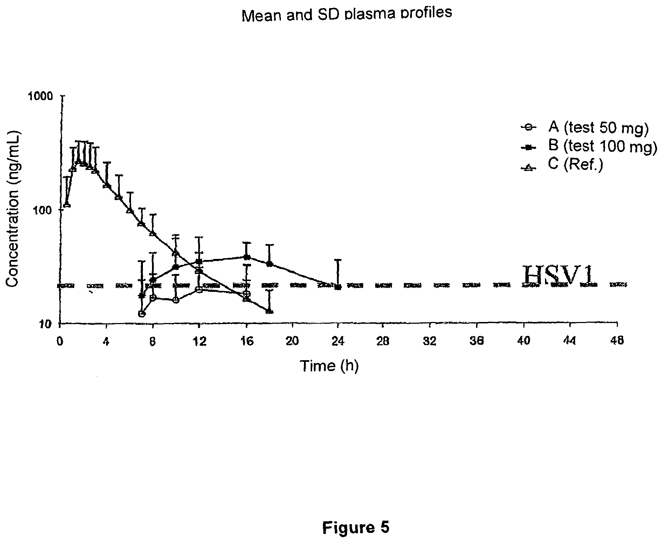

The plasma concentration profiles are presented in Figure 5.

The control tablet corresponding to the orally administered acyclovir exhibits an immediate-release profile, characterized by a rapid absorption phase, with a maximum concentration of 254 ng/ml at 1.5 hours. As seen in Figure 5, the control tablet allows the plasma concentration of acyclovir to remain higher than the minimal inhibitory concentration (MIC) during 14 hours.

To the contrary, the acyclovir bioadhesive slow release carriers of the present invention exhibit a sustained-release profile with a 6 hour delay in the absorption of the acyclovir, and a maximum concentration of 45.9 pg/ml at 12 hours. After an increasing absorption phase, mean plasma concentrations remain constant between 30.9 and 37.8 ng/ml for an 8 hour period. Additionally, the plasma concentration of acyclovir is now maintained above the minimal inhibitory concentration (MIC) during 16 hours. From the control tablet results, the relative bioavailability of the acyclovir released in the bioadhesive carrier could be calculated. This bioavailability was 35%, but for a dose that was two times less than the control. When calculated with the same dosage as the control, the bioavailability is 70%.

This example proves that the systemic transfer of acyclovir may occur by the transmucosal route, following the impregnation of the strong vascular oral mucosis or by oral route, following the swallowing of saliva enriched in solubilized acyclovir.

### EXAMPLE 5 - EVALUATION OF THE ACYCLOVIR SALIVA CONCENTRATION

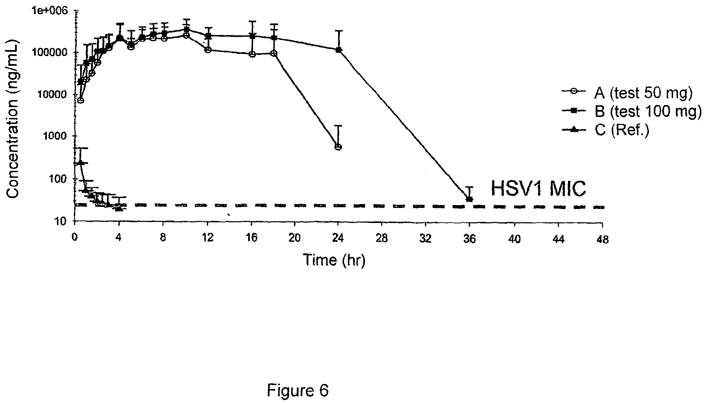

Figure 6 illustrates the acyclovir salivary concentration profiles obtained either with the control tablet or with the bioadhesive slow release carrier according to the present invention. As seen in Figure 6, when the control tablet is administered, the acyclovir appeared in the saliva around 30 minutes after administration, with a peak corresponding to a maximal concentration of 112 ng/ml. The acyclovir saliva concentration remains higher than the minimal inhibitory concentration (MIC) for 4 hours, but decreases quickly after the peak to become undetectable 10 hours after administration.

To the contrary, the bioadhesive slow release carrier of the present invention had very high levels of acyclovir saliva concentrations, even after the first sample taken at 30 minutes after administration. For instance, the saliva concentration of acyclovir was estimated at 6.8 µg/ml after administration of the 50 mg bioadhesive slow release carrier, and at 20 µg/ml after administration of the 100 mg bioadhesive slow release carrier after 30 minutes.

The acyclovir concentrations remained very high during 24 hours to 36 hours, with maximum concentration values of 387 µg/ml and 471 µg/ml respectively for the 50 and 100 mg bioadhesive slow release carriers. This demonstrated that the bioadhesive slow release carriers of the present invention permit the liberation of acyclovir very quickly (30 minutes) and for a long duration of time (36 hours) at the site of the herpes simplex virus-l. These concentrations are much more higher than the acyclovir minimal inhibitory concentration (MIC) required to treat herpes simplex virus-l, since they are respectively 17,000 (for the 50 mg carrier) to 21,000 (for the 100 mg carrier) times greater than the required minimal inhibitory concentration (MIC).

Furthermore, the bioadhesive carrier of the invention reaches a AUC / MIC (area under the curve/ minimal inhibitory concentration) ratio of 103,000 to 216,000 in local saliva, while the instant release carrier only provides an AUC / MIC ratio of 8. These exceptionally high ratios demonstrate the very high presence of acyclovir in the saliva, which is at a close proximity to the infection site and therefore favours considerably its local efficiency.

Taken together, these results demonstrate that the acyclovir bioadhesive slow release carrier of the invention favours a very early and sustained release of the acyclovir in the virus reservoir site. Furthermore, the very important amounts of acyclovir in saliva may contribute to limit intra and inter-individual contamination, since it is well known that the virus reservoir potential of saliva plays a key role in the viral spread.

### EXAMPLE 6 - EVALUATION OF THE ACYCLOVIR LABIAL CONCENTRATION

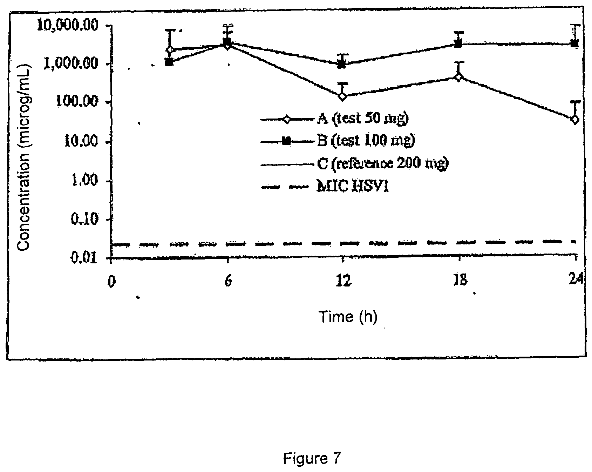

As disclosed in Figure 7, acyclovir is not detectable in the labial samples after administration of the control tablet.

To the contrary, when the acyclovir bioadhesive slow release carrier of the present invention was administered, the amount of acyclovir measured on the lips reached concentrations as high as 1 mg/ml. This strong presence of acyclovir on labial sites is maintained during at least 24h.

The results presented herein thus demonstrate that the carriers according to the invention favour the persistence of very high amounts of acyclovir on the lips i.e., at the expression site of the disease. This implies an increased pressure exerted against the HSV-1 virus, especially at the epidermic level, and suggests a greater efficiency of acyclovir against herpes labialis.

### EXAMPLE 7 - IN VIVO EVALUATION OF THE ADHESION-LASTING OF THE ACYCLOVIR BIOADHESIVE SLOW RELEASE CARRIER

To evaluate the adhesion time of the bioadhesive slow release carrier according to the present invention, this acyclovir carrier was applied on 12 healthy volunteers inside the upper lip. The presence of the carrier was checked at various times until 48 hours. The volunteers were checked on a regular basis for the loss of their carrier just until 24 hours after application. The results of this evaluation are disclosed in the following Table 1.

**Table 1- ADHESION TIME (hours)**

| | 50 mg carrier | 100 mg carrier |
|---|---|---|
| Median | 14 | 18 |
| Minimum | 6 | 1 0 |
| Maximum | 18 | 24 |

These results demonstrate that the carrier adhesion is completely compatible with a "once-daily" form of administration. Indeed, as long as the carrier remains at the application site, acyclovir is locally released in close proximity to the aimed infection site. Therefore, the acyclovir bioadhesive slow release carrier obtained with the method disclosed in Example 1 now renders possible the "once-daily" local administration of acyclovir, while achieving efficient loco-regional concentrations of the active principle with respect to the MIC.

## Claims

1. A prolonged release mucoadhesive buccal tablet comprising at least one acyclic guanosine antiviral agent, 1 to 75% by weight of a diluent, 1 to 10% by weight of an alkali metal alkylsulfate, 0.1 to 5% by weight of a binding agent, 5 to 80% by weight of at least one bioadhesive polymer selected from the group of natural polymers wherein said natural polymers are polysaccharides or, natural proteins from animal origin or vegetable origin or, synthetic polymers, and mixtures thereof and further comprising 5% to 80% by weight of at least one polymer that provides a sustained release of the at least acyclic guanosine antiviral agent for use for treating or preventing orofacial herpes, **characterized in that** only a single dose is administered.

2. A prolonged release mucoadhesive buccal tablet according to claim 1 for use according to claim 1, **characterized in that** orofacial herpes is frequently recurrent orofacial herpes.

3. A prolonged release mucoadhesive buccal tablet according to claim 1 for use according to claim 1 or 2 wherein the at least acyclic guanosine antiviral agent is in an amount of 10 mg to 200 mg.

4. A prolonged release mucoadhesive buccal tablet according to claim 3 for use according to claim 3 wherein the at least acyclic guanosine antiviral agent is in an amount of 50 mg to 200 mg.

5. A prolonged release mucoadhesive buccal tablet according to claim 4 for use according to claim 4 wherein the at least acyclic guanosine antiviral agent is in an amount of 50 mg.

6. A prolonged release mucoadhesive buccal tablet according to claim 1 for use according to claim 1 or 2 wherein the at least acyclic guanosine antiviral agent is acyclovir.

7. A prolonged release mucoadhesive buccal tablet according to claim 1 for use according to claim 1 or 2 wherein the prolonged release mucoadhesive buccal tablet comprises 2 to 6% by weight of an alkali metal alkylsulfate.

8. A prolonged release mucoadhesive buccal tablet according to claim 7 for use according to claim 1 or 2 wherein the alkali metal alkylsulfate is sodium lauryl sulfate.

9. A prolonged release mucoadhesive buccal tablet according to claim 1 for use according to claim 1 or 2 wherein the prolonged release mucoadhesive buccal tablet comprises 10 to 40% by weight of at least one bioadhesive polymer selected from the group of natural polymers wherein said natural polymers are polysaccharides or, natural proteins from animal origin or vegetable origin or, synthetic polymers, and mixtures thereof.

10. A prolonged release mucoadhesive buccal tablet according to claim 9 for use according to claim 1 or 2 wherein the at least one bioadhesive polymer is milk protein concentrate.

11. A prolonged release mucoadhesive buccal tablet according to claim 1 for use according to claim 1 or 2 wherein the prolonged release mucoadhesive buccal tablet comprises 10% to 40% by weight of at least one polymer that provides a sustained release of the at least acyclic guanosine antiviral agent.

12. A prolonged release mucoadhesive buccal tablet according to claim 11 for use according to claim 1 or 2 wherein the polymer that provides a sustained release is hypromellose.

13. A prolonged release mucoadhesive buccal tablet according to claim 1 for use according to claim 1 or 2 wherein the prolonged release mucoadhesive buccal tablet comprises 50 mg acyclovir, 15% by weight microcrystalline cellulose, 4.5% by weight sodium lauryl sulfate, 0.4% by weight polyvinylpyrrolidine, 20% by weight milk protein concentrate, 15% by weight hypromellose, 0.5% by weight of magnesium stearate and 0.4% by weight of colloidal silica.

## Patentansprüche

1. Mukoadhäsive bukkale Retardtablette, umfassend wenigstens einen antiviralen Wirkstoff aus azyklischem Guanosin, 1 bis 75 Gew.- eines Verdünnungsmittels, 1 bis 10 Gew.-% eines Alkalimetall-Alkylsulfats, 0,1 bis 5 Gew.-% eines Bindemittels, 5 bis 80 Gew.-% wenigstens eines bioadhäsiven Polymers, ausgewählt aus der Gruppe natürlicher Polymere, wobei es sich bei den natürlichen Polymeren um Polysaccharide oder natürliche Proteine tierischen oder pflanzlichen Ursprungs oder um synthetische Polymere und Mischungen daraus handelt, und darüber hinaus umfassend 5 bis 80 Gew.-% wenigstens eines Polymers, das eine verzögerte Freisetzung des wenigstens einen antiviralen Wirkstoffs aus azyklischem Guanosin zur Anwendung für die Behandlung oder Vorbeugung von orofazialem Herpes gewährleistet, **dadurch gekennzeichnet, dass** nur eine einzige Dosis verabreicht wird.

2. Mukoadhäsive bukkale Retardtablette nach Anspruch 1 zur Anwendung gemäß Anspruch 1, **dadurch gekennzeichnet, dass** es sich bei orofazialem Herpes um häufig rezidivierenden orofazialen Herpes handelt.

3. Mukoadhäsive bukkale Retardtablette nach Anspruch 1 zur Anwendung gemäß Anspruch 1 oder 2, wobei der wenigstens eine antivirale Wirkstoff aus azyklischem Guanosin in einer Menge von 10 mg bis 200 mg vorliegt.

4. Mukoadhäsive bukkale Retardtablette nach Anspruch 3 zur Anwendung gemäß Anspruch 3, wobei der wenigstens eine antivirale Wirkstoff aus azyklischem Guanosin in einer Menge von 50 mg bis 200 mg vorliegt.

5. Mukoadhäsive bukkale Retardtablette nach Anspruch 4 zur Anwendung gemäß Anspruch 4, wobei der wenigstens eine antivirale Wirkstoff aus azyklischem Guanosin in einer Menge von 50 mg vorliegt.

6. Mukoadhäsive bukkale Retardtablette nach Anspruch 1 zur Anwendung gemäß Anspruch 1 oder 2, wobei es sich bei dem wenigstens einen antiviralen Wirkstoff aus azyklischem Guanosin um Aciclovir handelt.

7. Mukoadhäsive bukkale Retardtablette nach Anspruch 1 zur Anwendung gemäß Anspruch 1 oder 2, wobei die mukoadhäsive bukkale Retardtablette 2 bis 6 Gew.-% eines Alkalimetall-Alkylsulfats umfasst.

8. Mukoadhäsive bukkale Retardtablette nach Anspruch 7 zur Anwendung gemäß Anspruch 1 oder 2, wobei es sich bei dem Alkalimetall-Alkylsulfat um Natriumlaurylsulfat handelt.

9. Mukoadhäsive bukkale Retardtablette nach Anspruch 1 zur Anwendung gemäß Anspruch 1 oder 2, wobei die mukoadhäsive bukkale Retardtablette 10 bis 40 Gew.-% wenigstens eines bioadhäsiven Polymers umfasst, ausgewählt aus der Gruppe natürlicher Polymere, wobei es sich bei den natürlichen Polymeren um Polysaccharide oder natürliche Proteine tierischen oder pflanzlichen Ursprungs oder um synthetische Polymere und Mischungen daraus handelt.

10. Mukoadhäsive bukkale Retardtablette nach Anspruch 9 zur Anwendung gemäß Anspruch 1 oder 2, wobei es sich bei dem wenigstens einen bioadhäsiven Polymer um Milchproteinkonzentrat handelt.

11. Mukoadhäsive bukkale Retardtablette nach Anspruch 1 zur Anwendung gemäß Anspruch 1 oder 2, wobei die mukoadhäsive bukkale Retardtablette 10 bis 40 Gew.-% wenigstens eines Polymers umfasst, das eine verzögerte Freisetzung des wenigstens einen antiviralen Wirkstoffs aus azyklischem Guanosin gewährleistet.

12. Mukoadhäsive bukkale Retardtablette nach Anspruch 11 zur Anwendung gemäß Anspruch 1 oder 2, wobei es sich bei dem Polymer, das eine verzögerte Freisetzung gewährleistet, um Hypromellose handelt.

13. Mukoadhäsive bukkale Retardtablette nach Anspruch 1 zur Anwendung gemäß Anspruch 1 oder 2, wobei die mukoadhäsive bukkale Retardtablette 50 mg Aciclovir, 15 Gew.-% mikrokristalline Zellulose, 4,5 Gew.-% Natriumlaurylsulfat, 0,4 Gew.-% Polyvinylpyrrolidin, 20 Gew.-% Milchproteinkonzentrat, 15 Gew.-% Hypromellose, 0,5 Gew.-% Magnesiumstearat und 0,4 Gew.-% kolloidales Siliziumdioxid umfasst.

## Revendications

1. Comprimé buccal muco-adhésif à libération prolongée comprenant au moins un agent antiviral de type guanosine acyclique, 1 à 75 % en masse d'un diluant, 1 à 10 % en masse d'un alkylsulfate de métal alcalin, 0, 1 à 5 % en masse d'un agent liant, 5 à 80 % en masse d'au moins un polymère bio-adhésif choisi dans le groupe constitué par les polymères naturels lesdits polymères naturels étant des polysaccharides, ou parmi les protéines naturelles d'origine animale ou végétale, ou parmi les polymères de synthèse, et leurs mélanges, et comprenant en outre 5 % à 80 % en masse d'au moins un polymère conférant une libération prolongée de l'au moins un agent antiviral de type guanosine acyclique pour utilisation dans le traitement prophylactique ou thérapeutique de l'herpès orofacial, **caractérisé en ce qu'**une seule dose est administrée.

2. Comprimé buccal muco-adhésif à libération prolongée selon la revendication 1 pour utilisation selon la revendication 1, **caractérisé en ce que** l'herpès orofacial est l'herpès orofacial à récurrence fréquente.

3. Comprimé buccal muco-adhésif à libération prolongée selon la revendication 1 pour utilisation selon la revendication 1 ou 2, où l'au moins un agent antiviral de type guanosine acyclique est présent à une teneur de 10 mg à 200 mg.

4. Comprimé buccal muco-adhésif à libération prolongée selon la revendication 3 pour utilisation selon la revendication 3, où l'au moins un agent antiviral de type guanosine acyclique est présent à une teneur de 50 mg à 200 mg.

5. Comprimé buccal muco-adhésif à libération prolongée selon la revendication 4 pour utilisation selon la revendication 4, où l'au moins un agent antiviral de type guanosine acyclique est présent à une teneur de 50 mg.

6. Comprimé buccal muco-adhésif à libération prolongée selon la revendication 1 pour utilisation selon la revendication 1 ou 2, où l'au moins un agent antiviral de type guanosine acyclique est l'acyclovir.

7. Comprimé buccal muco-adhésif à libération prolongée selon la revendication 1 pour utilisation selon la revendication 1 ou 2, où le comprimé buccal muco-adhésif à libération prolongée comprend 2 à 6 % en masse d'un alkylsulfate de métal alcalin.

8. Comprimé buccal muco-adhésif à libération prolongée selon la revendication 7 pour utilisation selon la revendication 1 ou 2, où l'alkylsulfate de métal alcalin est le laurylsulfate de sodium.

9. Comprimé buccal muco-adhésif à libération prolongée selon la revendication 1 pour utilisation selon la revendication 1 ou 2, où le comprimé buccal muco-adhésif à libération prolongée comprend 10 à 40 % en masse d'au moins un polymère bio-adhésif choisi dans le groupe constitué par les polymères naturels, où lesdits polymères naturels sont des polysaccharides, ou les protéines naturelles d'origine animale ou végétale, les polymères synthétiques et leurs mélanges.

10. Comprimé buccal muco-adhésif à libération prolongée selon la revendication 9 pour utilisation selon la revendication 1 ou 2, où l'au moins un polymère bio-adhésif est un concentré de protéine lactique.

11. Comprimé buccal muco-adhésif à libération prolongée selon la revendication 1 pour utilisation selon la revendication 1 ou 2, où le comprimé buccal muco-adhésif à libération prolongée comprend 10 % à 40 % en masse d'au moins un polymère conférant une libération prolongée de l'au moins un agent antiviral de type guanosine acyclique.

12. Comprimé buccal muco-adhésif à libération prolongée selon la revendication 11 pour utilisation selon la revendication 1 ou 2, où le polymère conférant une libération prolongée est l'hypromellose.

13. Comprimé buccal muco-adhésif à libération prolongée selon la revendication 1 pour utilisation selon la revendication 1 ou 2, où le comprimé buccal muco-adhésif à libération prolongée comprend 50 mg d'acyclovir, 15 % de cellulose microcristalline, 4,5 % en masse de laurylsulfate de sodium, 0,4 % en masse de polyvinylpyrrolidine, 20 % en masse de concentré de protéine lactique, 15 % en masse d'hypromellose, 0,5 % en masse de stéarate de magnésium et 0,4 % en masse de silice colloïdale.
